# NEUE EUROPÄISCHE PATENTSCHRIFT

(11) **EP 1 459 701 B2**
(45) Veröffentlichungstag und Bekanntmachung des Hinweises auf die Entscheidung über den Einspruch: **03.03.2021**
(45) Hinweis auf die Patenterteilung: 02.05.2007
(21) Anmeldenummer: 03006052.9
(22) Anmeldetag: 19.03.2003
(51) Int. Cl.: A61C 9/00, A61K 6/00, A61K 6/10

(54) **Dentalset für die Sulkus-Retraktion**
Dental set and method for widening the gingival sulcus
Ensenmble dentaire et procédé pour élargir le sillon gingival

(43) Veröffentlichungstag der Anmeldung: 22.09.2004
(73) Patentinhaber: Coltène/Whaledent AG, 9450 Altstaetten (CH)
(72) Erfinder: Lampl, Stephan, 9450 Lüchingen (CH); Kollefrath, Ralf, Dr., 9463 Oberriet (CH); Lübbers, Dierk, Dr., 9450 Altstätten (CH)
(74) Vertreter: Hepp Wenger Ryffel AG

(56) Entgegenhaltungen:
- DE-A- 3 737 552
- US-A- 4 677 139
- US-A- 5 676 543
- Precisione e Contorno nella Ricostruzione Prostesica (1987)

## Beschreibung

Die Erfindung betrifft die Verwendung eines Dentalsets zur Freilegung des Zahnhals-Bereich im Rahmen einer Abformung der Gebiss-Situation.

Für die Erstellung von Zahnersatz, insbesondere bei Zahn-Brücken muss dem Zahntechniker ein Abdruck der individuellen Situation der betroffenen Zähne bzw. des benachbarten Kieferabschnittes vorliegen. Hierzu wird deshalb vom Zahnarzt eine Abformung dieser Gebiss-Situation vorgenommen. Um den Übergang vom Zahnersatz zum Kiefer möglichst zu verdecken, ist es erforderlich, den Zahnhals freizulegen, welcher durch das Zahnfleisch verdeckt wird. Deshalb muss bei der Erstellung des Gebiss-Abdrucks dieser Bereich, d.h. der Sulkus freigelegt werden.

Neben einem weit verbreiteten Verfahren, bei dem ein sogenannter Retraktionsfaden in den Bereich des Sulkus eingebracht wird, beschreibt US 5,676,543 einen weiteren Weg. Gemäss dieser Schrift wird mit einer härtbaren Formmasse ein Abdruck der Gebiss-Situation erstellt. Die ausgehärtete Form wird nachfolgend entfernt. In diese Form wird eine Schicht eines spritzfähigen und härtbaren Materials aufgebracht und die so präparierte Form erneut in den Mund eingesetzt. Das spritzfähige und härtbare Material muss im weiteren zwingend eine blutstillende oder Gewebe-Kontraktion verursachende Verbindung enthalten. Durch Druckanwendung auf die Form wird dieses härtbare Material in den Bereich zwischen Zahnhals und Zahnfleisch gepresst. Durch das blutstillende Material wird eine Gewebekontraktion verursacht. Der Sulkus zieht sich vom Zahnhals zurück. Nach dem Säubern kann eine genaue Abformung der Zahnsituation einschliesslich des freigelegten Sulkus erfolgen.

Es hat sich allerdings herausgestellt, dass das Einbringen der spritzfähigen, härtbaren Material in die negative Gebiss-Abformung nicht immer zu den gewünschten Resultaten führt. Teilweise wird beim Einsetzen der Abformung ein Teil des spritzfähigen Materials verschmiert, so dass eine korrekte Sulkus-Retraktion nicht mehr gewährleistet ist. Schliesslich ist auch die Kontraktion des Sulkus durch das blutstillende Mittel nicht immer ausreichend, um die Abdruckqualität zu gewährleisten. Im weiteren gestaltet sich auch die mengenmässige Applikation des spritzfähigen Materials schwierig, da beim Auftragen die Konturen der Negativ-Form verdeckt werden und somit keine Kontrolle der Schichtdicke mehr möglich ist.

Die Aufgabe der Erfindung ist es, die Nachteile des Bekannten zu vermeiden, insbesondere eine alternative Methode zur Freilegung des Zahnhalses bereitzustellen.

Dies wird durch die Verwendung eines Dentalsets gemäss dem kennzeichnenden Teil des unabhängigen Anspruchs gelöst.

Das Verfahren weist eine Reihe von aufeinanderfolgenden Schritten auf. Mit einer aushärtbaren Verbindung wird zuerst ein Gebiss-Abdruck hergestellt, welcher nach dessen Aushärtung entnommen wird. In den Grenzbereich zwischen Zahn und Zahnfleischrand wird mit einem geeigneten Instrument ein Silikonmaterial aufgetragen. Das kennzeichnende Merkmal dieses Silikonmaterials ist dessen Volumenexpansion bei der Aushärtung. Der zuvor hergestellte Gebiss-Abdruck wird wieder auf die Zähne aufgesetzt, wodurch dieser Abdruck eine einseitige Begrenzung für die Expansion des Silikonmaterials bildet. Die Expansion des Silikons kann nur in Richtung des Sulkus erfolgen, so dass dieser vom Zahnhals abgelöst wird. Der Sulkus wird also vom Zahnhals gelöst und nicht nur durch Gewebekontraktion vom Zahnhals zurückgezogen.

Es sind verschiedene Material-Systeme bekannt, welche für die Herstellung des Gebiss-Abdrucks geeignet sind. In der Regel bestehen diese Systeme aus mindestens einer Silikonverbindung sowie einem Härtungs-Katalysator. Vorzugsweise werden additionsund kondensationsvernetzende Silikonverbindungen eingesetzt. Die beiden Komponenten für die Abformmasse können entweder getrennt vorliegen und erst unmittelbar vor der Anwendung gemischt werden oder sie bilden eine Mischung, welche durch Feuchtigkeit oder ähnliches zur Aushärtung gebracht wird.

Das bei der Aushärtung expandierende Silikonmaterial kann aus einer einzigen Silikonverbindung oder einem Gemisch verschiedener Silikonverbindungen bestehen. Dieses expandierende Silikonmaterial weist eine minimale Expansion von mindestens 20 %, weiter bevorzugt von mindestens von 35 %, besonders bevorzugt von mindestens 50 % und meist bevorzugt von mindestens 70 % bezogen auf das ursprüngliche Volumen auf. Die Werte für diese Volumenexpansion beziehen sich hierbei auf die Expansion des Material in einem nicht begrenzten, d.h. offenen Volumen. Der Begriff expandierend ist im Rahmen der Erfindung so zu verstehen, dass die Volumenexpansion während oder nach der Härtungsreaktion des Silikonmaterials bzw. eines Gemisches aus mehreren Silikonverbindungen erfolgt. Als Härtungsreaktion sind Reaktionsabläufe zur verstehen, welche zur Ausbildung von neuen inter- oder intramolekularen Verknüpfungen führen. Die Expansion wird in der Regel verzögert nach dem Beginn dieser Reaktionsabläufe einsetzen und über das Ende dieser Reaktionen hinaus andauern. Geeignete Silikonverbindung, welche ein solches Expansionsverhalten zeigen, sind additionsvernetzende Silikonverbindungen. Ein expandierendes Silikonmaterial mit den zuvor angegebenen Expansionsvolumen erlaubt eine verbesserte und einfachere Steuerung der Sulkus-Retraktion. Gegebenenfalls wird vor der Auftragung des Silikonmaterials ein Härtungskatalysator zugemischt oder das Silikonmaterial enthält bereits einen Katalysator, der die Aushärtung aufgrund der vorhandenen Feuchtigkeit auslöst. Neben dem spezifischen Expansionsverhalten kann auch durch die aufgetragene Menge steuernd eingegriffen werden. Beide Faktoren beeinflussen daher gegenseitig das Ergebnis der Retraktion. Vorteilhaft ist ein additionsvernetzendes, expandierendes Silikonmaterial, welches als Zwei-Komponentensystem eingesetzt wird. Die als wesentlichen Bestandteile der beiden Komponenten verwendeten unterschiedlich funktionalisierten Poly(dimethyl)siloxane, beispielsweise Dihydroxy- oder Divinyl-Poly(dimethyl)siloxane weisen eine Viskosität vorzugsweise zwischen 5 und 100 Pa.s auf. Die beiden Komponenten enthalten weiterhin Füllstoffe, die üblicherweise für Dentalmassen eingesetzt werden. Diese Füllstoffe können entweder oberflächenbehandelt oder ohne Oberflächenbehandlung sein. Beispiele für Füllstoffe sind Kieselerde, pyrogene Kieselsäure, Calciumcarbonat, Quarzmehl oder Silicate.

Die Verwendung additionsvernetzender Silikone vermeidet mögliche gesundheitliche Beeinträchtigungen des Patienten, da bei der Aushärtung keine nachteiligen Verbindungen freigesetzt, d.h. abgespalten werden. Im weiteren besitzen diese Art von Verbindungen den Vorteil, dass sie sich nicht mit dem Abformungsmaterial verbinden. Nach der Aushärtung des expandierenden Silikons kann dieses entweder in einem Stück oder in wenigen Teilstücken vom Zahnbereich entfernt werden. Gleichzeitig entsteht für den Zahnarzt die Möglichkeit, den Erfolg der Retraktion zu kontrollieren, gegebenenfalls erneut expandierendes Silikonmaterial an die ungenügend freigelegte Stelle aufzubringen und den Retraktionsvorgang zu wiederholen. Diese Vorgehensweise ist mit einer Silikonverbindung, welche sich dauerhaft an die Abformung bindet, nicht möglich. Hier wäre eine vollständige Wiederholung der gesamten Prozedur erforderlich.

Weiter vorteilhaft ist das Verfahren, wenn vor der Auftragung des expandierenden Silikonmaterials auf den Grenzbereich zwischen Zahn und Zahnfleisch mindestens eine blutungsstillende Verbindung bzw. ein Adstringend aufgetragen wird. Beispielhafte blutungsstillende Verbindungen oder Adstringens sind einschliesslich der verschiedenen, geeigneten Hydrate Kaliumaluminiumsulfat, Aluminiumsulfat, Aluminiumeisensulfat, Aluminiumammoniumsulfat, Eisenchlorid, Aluminiumchlorid, Natriumchlorid, Zinkchlorid, Zinkphenolsulfat, Gerbsäuren, Adrenalin oder weitere bekannte Verbindungen. Durch die Auftragung dieser Verbindungen können bei der Expansion des Silikonmaterials entstehende Blutung unmittelbar zum Stehen gebracht werden. Somit lässt sich der verdrängte Sulkus nach Entfernen des ausgehärteten und expandierten Silikons einfach von ausgetretener Flüssigkeiten reinigen und bleibt für eine nachfolgende Detailabformung der Gebisssituation weitgehend frei von weiteren Flüssigkeitsaustritten. Da meist eine chemische Kompatibilität der blutungsstillenden Verbindung mit dem expandierenden Silikon nicht gewährleistet ist, müssen deshalb beide Komponenten getrennt aufgetragen werden. Einzelne blutstillende Verbindungen wie beispielsweise Gerbsäuren lassen sich allerdings in das additionsvernetzende, expandierende Silikonmaterial einarbeiten, so dass eine gemeinsame Auftragung auf den Bereich zwischen Zahn und Zahnfleisch möglich ist.

Ebenfalls vorteilhaft ist das Verfahren, wenn der wiedereingesetzte Gebiss-Abdruck, d.h. die Abformung durch den gegenüberliegenden Zahnbogen in seiner Position gehalten wird. Der Patient kann dies durch einfaches Zubeissen erreichen. Ein umständliches und auch Fehler verursachendes Halten der Abformung durch den Zahnarzt kann entfallen. Aufgrund der Fixierung durch die gegenüberliegende Zähne ist eine gleichmässige Retraktion des Sulkus gewährleistbar.

Das Dentalset wird zur Ausführung des oben ausgeführten erfindungsgemässen Verfahrens eingesetzt. Das Set besteht aus einer härtbaren Abformmasse, mit welcher ein Gebiss-Abdruck herstellbar ist, und mindestens einem expandierendem Silikonmaterial, wobei die Volumenexpansion mindestens 20 %, vorzugsweise mindestens 35 %, weiter bevorzugt 50 % und besonders bevorzugt 70 % gegenüber dem ursprünglichen Volumen der nicht ausgehärteten Mischung beträgt.

Weiter vorteilhaft enthält dieses Set eine blutungsstillende Verbindung analog den bereits vorstehend beschriebenen, welche separat auf die zu behandelnde Stelle aufzutragen ist.

Die Erfindung wird im folgenden anhand von Beispielen und der Figur näher erläutert.

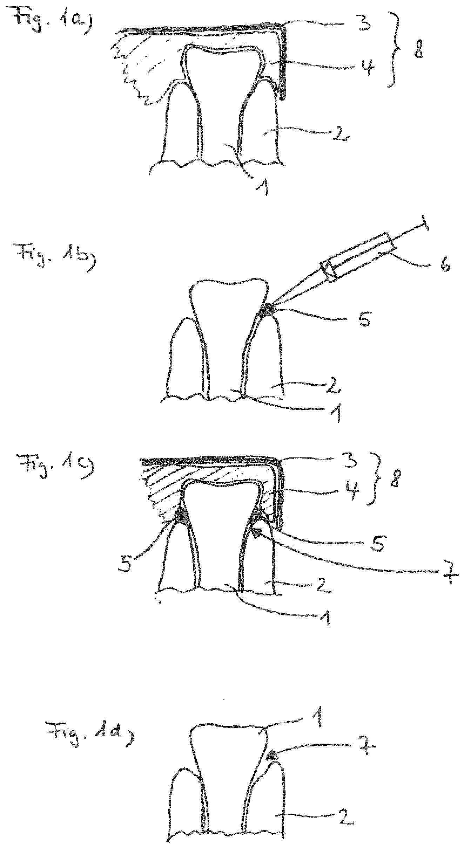

- Figuren 1a bis d:: Darstellung der wesentlichen Verfahrensschritte

### Beispiel 1:

Die Abfolge an Schritten für die Sulkusretraktion beginnt mit der Herstellung einer Gebiss-Abformung wie dies in Figur 1a dargestellt ist. Der Zahn 1 sowie das umgebende Zahnfleisch wird der Abformmasse 3 umhüllt. Als Abformmasse wird eine kondensationsvernetzende Knetmasse Speedex Putty (Fa. Coltène AG) eingesetzt, welche hierzu in einen Formlöffel 4 eingebracht wird. Der Formlöffel 4 wird auf den Zahn 1 bzw. das umgebende Gewebe 2 gedrückt.

Nach der Aushärtung des Abformmasse 3, welche nach etwa 3 bis 4 Minuten erfolgt ist, kann die Abformung 8 entfernt werden und der Zahn 1 sowie das Gewebe 2 gereinigt werden. Bevor im weiteren die expandierende Silikonverbindung aufgebracht wird, erfolgt ein Aufpinseln von Aluminiumsulfat als blutungsstillender Verbindung. In den Grenzbereich zwischen Zahn 1 und dem Gewebe 2 wird mit einem in-situ Mischapplikator 6 die expandierende Silikonverbindung 5 eingespritzt. Diese expandierende Silikonverbindung 5 besteht aus zwei Komponenten A und B.
Die Komponente A setzt sich zusammen aus:

| | |
|---|---|
| 10g | alpha, omega-Dihydroxy-Polydimethylsiloxan (Viskosität 18 Pa.s; Wacker Silicones) |
| 5g | Quarzmehl Sikron B600 (Quarzwerke Frechen, D) |
| 0.05g | Silopren U-Katalysator Pt/D (GE Bayer Silicones) |
| 0.02g | Divinyltetramethyldisiloxan (Fluka) |

Die Komponente B weist folgende Zusammensetzung auf:

| | |
|---|---|
| 9.8g | alpha, omega-Divinyl-polydimethylsiloxan (Viskosität 20 Pa.s; Wacker Silicones) |
| 5g | Quarzmehl Sikron B600 (Quarzwerke Frechen, D) |
| 0.3g | Polymethylhydrosiloxan (Viskosität 20 mPa.s; Wacker Silicones) |

Beide Komponenten A und B werden im Masseverhältnis 1:1 homogen gemischt. Durch das unmittelbares Mischen der Silikonkomponenten A und B beim Auftragen wird die Aushärtung gestartet. Die Oberfläche der erstellten Abformung 8 wird vor dem Aufsetzen mit einem Trennmittel behandelt und wird nach dem Auftragen der expandierenden Silikonverbindung wieder auf den Zahn aufgesetzt (Figur 1c). Damit ist die Expansion des homogen Gemisches der Silikonkomponenten A und B in der Ausdehnungsrichtung begrenzt und kann nur auf den Sulkusbereich 7 wirken. Die getrennte Auftragung des Aluminiumsulfats und Gemisches ist aufgrund der Unverträglichkeit beider Komponenten erforderlich.

Nach der erfolgten Expansion des Silikon-Komponentengemisches 5 wird die Abformung 8 abgenommen. Das expandierte Silikon-Komponentengemisch 5 verbleibt getrennt von der Abformung 8 am Zahn 1 und kann in wenigen, zusammenhängenden Stücken aus dem nun erweiterten Sulkusbereich 7 entfernt werden. Die endgültige Abformung der Zahnsituation kann nach einer Reinigung des erweiterten Sulkus mit einer hohen Präzision vorgenommen werden. Figur 1d zeigt den Zahn 1 mit dem freigelegten Sulkus 7 und dem darunter liegenden Zahnfleisch 2.
Im Falle einer ungenügenden Freilegung des Sulkus können die Verfahrensschritte, wie sie in den Figuren 1b und 1c skizziert dargestellt sind, wiederholt werden.

Gemäss dem zweiten Beispiel wird die Gebiss-Abformung mit einer additionsvernetzenden Knetmasse AFFINIS Putty (Fa. Coltène AG) hergestellt. Die Knetmasse wird dazu in einen Formlöffel eingebracht und dieser auf das Gebiss aufgesetzt. Nach der Aushärtung des Material wird die Abformung entfernt und nach einer Reinigung mit einem Trennmittel GI Mask Separator (Fa. Coltène AG) versehen. Aufgrund des Trennmittels können die Abformung und die ausgehärtete, expandierende Silikonverbindung getrennt voneinander entfernt werden.
Die Sulkus-Retraktion erfolgt weiter wie im Beispiel zuvor beschrieben.

Die endgültige Abformung der Zahnsituation kann mit den handelsüblich zur Verfügung stehenden Abformmassen durchgeführt werden.

## Patentansprüche

1. Verwendung eines Dentalsets geeignet für eine Sulkus-Retraktion, enthaltend eine härtbare Abformmasse (3) für die Erstellung einer Abformung (8), insbesondere einer Gebiss-Abformung (8), und mindestens eine härtbare Silikonverbindung (5), **dadurch gekennzeichnet, dass** die Silikonverbindung (5) bei der Aushärtung eine Volumenexpansion von mindestens 20 %, vorzugsweise mindestens 35 % gegenüber dem ursprünglichen Volumen der nicht gehärteten Verbindung aufweist, wobei
mit der härtbaren Abformmasse (3) zuerst ein Gebiss-Abdruck hergestellt wird, welcher nach dessen Aushärtung entnommen wird,
in den Grenzbereich zwischen Zahn und Zahnfleischrand das Silikonmaterial (5) aufgetragen wird,
und der zuvor hergestellte Gebiss-Abdruck wieder auf die Zähne aufgesetzt wird,
wodurch dieser Abdruck eine einseitige Begrenzung für die Expansion des Silikonmaterials (5) bildet, sodass die Expansion des Silikonmaterials (5) nur in Richtung des Sulkus erfolgen kann und dieser vom Zahnhals abgelöst wird.

2. Verwendung nach Anspruch 1, **dadurch gekennzeichnet, dass** das Set eine blutungsstillende Verbindung enthält.

3. Verwendung nach Anspruch 1 oder 2, **dadurch gekennzeichnet, dass** die härtbare Abformmasse (3) mindestens eine weitere Silikonverbindung und einen Härtungskatalysator enthält.

4. Verwendung nach einem der Ansprüche 1 bis 3, **dadurch gekennzeichnet, dass** die härtbare und expandierende Silikonverbindung (5) aus der Gruppe der additionsvernetzenden Silikonverbindungen ausgewählt ist.

## Claims

1. Use of a dental set suitable for sulcus retraction, containing a curable impression material (3) for the production of an impression (8), in particular a denture impression (8), and at least one curable silicone compound (5), **characterised in that** the silicone compound (5) exhibits a volume expansion of at least 20%, preferably at least 35%, on curing compared with the original volume of the non-cured compound, wherein
a denture impression is first made with the curable impression compound (3), which is removed after it has cured,
the silicone material (5) is applied to the boundary region between the tooth and the gingival margin,
and the previously made denture impression is placed on the teeth again,
whereby this impression forms a one-sided limitation for the expansion of the silicone material (5), so that the expansion of the silicone material (5) can only take place in the direction of the sulcus and this is detached from the tooth neck.

2. Use according to claim 1, **characterized in that** the set contains a haemostatic compound.

3. Use according to claim 1 or 2, **characterised in that** the curable impression compound (3) contains at least one further silicone compound and a curing catalyst.

4. Use according to any one of claims 1 to 3, **characterized in that** the curable and expanding silicone compound (5) is selected from the group of addition-curing silicone compounds.

## Revendications

1. Utilisation d'un ensemble dentaire approprié pour la rétraction du sillon, contenant un matériau d'empreinte durcissable (3) pour la réalisation d'une empreinte (8), en particulier d'une empreinte de prothèse dentaire (8), et au moins un composé de silicone durcissable (5), **caractérisée en ce que** le composé de silicone (5) présente, lors du durcissement, une expansion volumique d'au moins 20 %, de préférence d'au moins 35 %, par rapport au volume initial du composé non durci, dans laquelle
une empreinte de prothèse dentaire est d'abord réalisée avec le matériau d'empreinte durcissable (3), qui est retiré après avoir durci,
le matériau en silicone (5) est appliqué sur la zone limite entre la dent et le bord gingival,
et l'empreinte de la prothèse dentaire précédemment réalisée est à nouveau placée sur les dents,
cette empreinte forme une limitation unilatérale de l'expansion de la matière silicone (5), de sorte que l'expansion de la matière silicone (5) ne peut avoir lieu que dans la direction du sillon et que celui-ci se détache du collet de la dent.

2. Utilisation selon la revendication 1, **caractérisée en ce que** le kit comprend un composé hémostatique.

3. Utilisation selon la revendication 1 ou 2, **caractérisée en ce que** le composé d'empreinte durcissable (3) contient au moins un autre composé de silicone et un catalyseur de durcissement.

4. Utilisation selon l'une quelconque des revendications 1 à 3, **caractérisée en ce que** le composé de silicone durcissable et expansible (5) est choisi dans le groupe des composés de silicone durcissant par addition.
